Europäisches Patentamt

European Patent Office

Office européen des brevets

(11) Publication number : **0 511 932 A2**

## (12) EUROPEAN PATENT APPLICATION

(21) Application number : 92500045.7

(22) Date of filing : 24.04.92

(51) Int. Cl.⁵ : **C08B 37/00,** C08B 37/04, C08B 37/14, A61K 31/725

(30) Priority : 27.04.91 ES 9101063

(43) Date of publication of application :
04.11.92 Bulletin 92/45

(84) Designated Contracting States :
AT BE CH DE DK FR GB GR IT LI LU MC NL PT SE

(71) Applicant : **LABORATORIOS ANDROMACO S.A.**
**Azcona 31**
**E-28028 Madrid (ES)**

(72) Inventor : **Tuduri Taviel De Andrade, Pilar**
**San Rosendo 1 dupl**
**E-28022 Madrid (ES)**
Inventor : **Pivel Ranieri, Juan Pablo**
**San Rosendo 1 dupl**
**E-28022 Madrid (ES)**
Inventor : **Guerrero-Gomez Pamo, Antonio**
**San Rosendo 1 dupl**
**E-28022 Madrid (ES)**
Inventor : **Real Gallego, Almudena**
**Avda. Complutense 22**
**E-28040 Madrid (ES)**
Inventor : **Guenchea Amurrio, Guillermo**
**Avda. Complutense 22**
**E-28040 Madrid (ES)**
Inventor : **Bueren Roncero, Juan Antonio**
**Avda. Complutense 22**
**E-28040 Madrid (ES)**
Inventor : **Maganto Fernandez, Gabriel**
**Avda. Complutense 22**
**E-28040 Madrid (ES)**

(74) Representative : **Garcia Cabrerizo, Francisco**
**OFICINA GARCIA CABRERIZO S.L. Vitruvio 23**
**E-28006 Madrid (ES)**

(54) **Process for the obtaining of polymers with activity on the hematopoietic system.**

(57)    Described herein is a process for the obtaining of polymers which are of polysaccharide character with activity upon the hematopoietic system, said polymers being obtained from polycarboxylated polysaccharides such as alginic acid, gum arabic or gum tragacanth of diverse biological sources, which process consists in the ultrasonic irradiation of well-defined aqueous suspensions of said polymers under specified conditions of amplitude, frequency, time and temperature, after which the products thus obtained are purified by means of exhaustive dialysis and are given the form of solids by means of lyophilization or spray-drying, the products themselves being endowed with an effect upon the activity of the hematopoietic system. The process has applications in the pharmaceutical industry.

EP 0 511 932 A2

The present invention relates to a process for obtaining a family of molecules which are of distinct molecular weight and polysaccharide in character, which molecules are to be obtained from polycarboxylated polysaccharides and the derivatives thereof such, for example, as alginic acid, gum arabic or tragacanth, said molecules being of diverse biological origins and endowed with an activity that causes stimulation and recovery of the hematopoietic system, which activity renders them particularly useful in clinical situations involving suppresion of bone marrow, such as occur following chemotherapy and/or radiation treatment for neoplastic conditions. Hematopoiesis-stimulating activity is well documented for various molecules of polysaccharide nature, Some of these molecules of polysaccharide nature, which have been described as exerting this activity, are: glucan P., which is obtained from the cell wall of Saccharomyces cerevisiae, a yeast; "Krestin" which is obtained from the fungus Cariolus versicolor; "Lentinan", obtained from the fungus Lentinus eodes; "Schizopullan", obtained from the fungus Schyzophyllum commune; and others (M.L. Patchen et al., J. Biol. Resp. Mod. 5:45 (1986); M.L. Patchen et al., J. Biol. Resp. Mod. 3:627 (1984). Also, for some of the polycarboxylic polysaccharide molecules, activities applicable to industry have been described, as, for example, in the case of alginic acid and alginates, gum arabic and gum tragacanth. With respect to the alginates, their field of application within the context of pharmaceutical and para-pharmaceutical products is quite extensive and comprises such areas as antacid/anti-reflux activity, use as excipients in galenical formulations and as components in systems for the controled release of drugs, cicatrizing activity, and as additives in cosmetic products and those for dental use. Alginates have likewise been utilized for the immobilization of enzymes (beta-glucosidase), as additives in food products, and also as support for microbiological cultures and for culturing the cells of animal tissues. Gum arabic and gum tragacanth have been employed in the pharmaceutical and food-processing industries as mucilaginous binders, as excipients for tablets, as emulsifiers, as stabilizers for colorants and as flavoring agents. However, despite this abundant scientific literature, which relates both to the biological activity of polysaccharides and to that of the alginates, gum arabic and tragacanth in particular, there has been no description to date of any activity of these compounds and/or of their derivatives on the hematopoietic system.

On the other hand, there have been described in tile scientific literature a wide range of methods that are utilized for the partial depolymerization of polysaccharides, among which methods we may mention --insofar as they relate to the present invention-- the depolymerization of bacterial polysaccharides by means of ultrasonic radiation and that of alginates, in particular, due to the effect of ionizing radiations. Proceeding from the previous studies, both pharmacologic and methodological, it is possible to infer the novelty of the present invention, which consists of the depolymerization of polycarboxylated polysaccharides by means of ultrasonic irradiation under controled and fixed conditions for the purpose of obtaining partially depolymerized water-soluble derivatives which are nontoxic and endowed with an activity that serves to stimulate hamatopoiesis or to bring about the recovery thereof in situations of hematologic impairment.

## General Description of the Process

The process offered by the present invention consists in the obtaining of diverse derivatives of polycarboxylated polysaccharides of diverse biological origin, whereby the starting substances are depolymerized by process of ultrasonic irradiation. The diverse products are obtained by varying the time of ultrasonic irradiation under fixed and controled conditions. Among the natural sources of alginic acid, we consider algae such as the laminariaceae, for example, Laminariacea digitada (L.), the lesioniaceae, for example, Macrocystis pyrifera, and bacteria of the genus Pseudomonas, as exemplified by Pseudomonas aeruginosa, and those of the genus Azotobacter Vinelandii or by Azotobacter chroocoocum. Among, the natural sources of gum arabic, we consider the various species of acacia trees (of the leguminosae family), as, for example, Acacia senegal (L.) or Acacia verek. And among the natural source of gum tragacanth, there are the various species of Astragalus (of the leguminosae family), such as, for example, Astragalus gummifer Labill.

The phases of the process are described hereinunder:

1.- In the first stage: an aqueous suspension is prepared of the starting product, in quantities of between 5 and 25 mg/ml in a final volume of distilled water, which final volume may range between 10 and 200 ml.

2.- In the second stage; the suspension described in the first stage is subjected to ultrasound, for which purpose an "MSE Soniprep 150" device is utilized under the following conditions:

- Titanium probe suitable for the volume of sample.
- Amplitude: 5 - 15 μm.
- Frecuency of 23 KHz.
- The ultrasonic treatment is applied for a length of time that ranges between 0.5 and 8 hours. During the process, the ultrasonic treatment is performed in receptacles which are kept refrigerated by the recirculation of a coolant liquid, in such a way that the temperature is maintained within the range of 0°-15° C. Given the conditions of temperature, the control of oxidation by bubbling with $N_2$ is not necessary.

3.- In a third stage: the solutions obtained in the preceding stage are subjected to a procedure of cold-chamber (4°-8° C) exhaustive dialysis against distilled water during 48 hours, which procedure is effected in a Visking dialysis bag, with a molecular cut-off of between 10,000 and 14,000 daltons, said solutions having been previously washed with hot $H_2O$ and distilled water in copious amounts. Changes are effected in the dialysis liquid every 24 hours. The volumetric ratio between the dialyzed liquid and the dialyzing liquid is on the order of 0.01.

4.- In a fourth stage; the dialyzed liquid is dehydrated by lyophilization or by spray-drying, whereby the final product thus obtained is a yellowish-white, mildly bright powder of cottony appearance. The products obtained according to the process described are nontoxic, and they exhibit a series of pharmacological properties that are the basis of their therapeutic usefulness in human and veterinary medicine.

Toxicity studies:

The product obtained according to the process described in example 1, when administered to $F_1$ (C57/Bl x Balb/C) mice in the range of 10 - 60 mg/kg$^{-1}$ as a single intravenous dose, proved to be nontoxic, both by an evaluation of mortality 10, 14, 20 or 30 days after administration, and by the histological analyses of various tissues (liver, spleen, kidney, beart, lung, entothelium and mesenteric ganglia) at the above-indicated time intervals.

Increase in hematopoietic activity, as evaluated in terms of the number of CFU-GM spleen precursor cells:

The administration to $F_1$ (C57/Bl x Balb/C) mice of the product obtained according to the process described in example 1, given as a single dose of 60 mg/kg by the intravenous route, causes an increase in the number of CFU-GM spleen colonies. The kinetics of this stimulation presents peaks on the 5th and 15th days following administration of the product, with levels of the number of colonies attaining to between 300% and 400% as compared to the basal levels. Such activation of the hematopoietic response persists 30 days after administrarion of the product, with levels of the number of CFU-GM colonies being increased by about 500% over the basal level. Likewise, the increase in the number of CFU-GM spleen colonies, when evaluated on the 5th day post-treatment, presents a dosage/effect relationship in the range of 10 - 100 mg/kg.

Increase in hematopoietic activity, as evaluated in terms of the number of BFU-E spleen precursor cells:

The administration of the product obtained according to the process described in example 1, to $F_1$ (C57/Bl x Balb/C) mice, under the same conditions as those described in the preceding paragraph, provokes an increase in the number of BFU-E colonies with peaks on the 5th day (250% over the basal level) and the 15th day (250% over the basal level) following said administration, while likewise presenting a stimulation which persists for 30 days after the day when the product was administered. Administration of the product at a dosage level of 60 mg/kg entails, as a consequence, a slight increase in splenic celullarity (on the order of 50%), which increase is maintained for 30 days after such administration.

Protective effect in mice subjected to lethal irradiation (7.6 Gy):

The administration of the product obtained according to the process described in example 1, in a single intravenous dose of 60 mg/kg to $F_1$ (C57/Bl x Balb/C) mice, which are also subjected to a lethal dose of irradiation (7.6 Gy), resulted in total protection as evaluated in terms of survival 30 days after said irradiation, when the product was administered some hours thereafter. When the product is administered one hour prior to irradiation, the protection is on the order of 90%.

Restorative effect on splenic and medullary hematopoietic activity in $F_1$ (C57/Bl x Balb/C) mice subjected to sublethal irradiation (3 Gy):

The administration of the product obtained according to the process described in example 1, at a dosage level of 60 mg/kg, gave rise to a noteworthy restorative effect on hematopoietic activity as evaluated in terms of the cellularity (femur and spleen) and the number of CFU-GM colonies (femur and spleen). When said product is administered one day prior to irradiation, the cellularity of femur and spleen presents increases of 30% and 55%, respectively, over the control values; and the number of colonies in the femur and spleen presents increases on the order of 27% and 980%, respectively, over the control values. When the product was administered one hour after irradiation, the increases of cellularity in femur and spleen were on the order of 60% and 70%, respectively, and the increases in the number of CFU-GM colonies in the femur and spleen were on the order of 75% and 2500%, respectively.

Analytical Data:

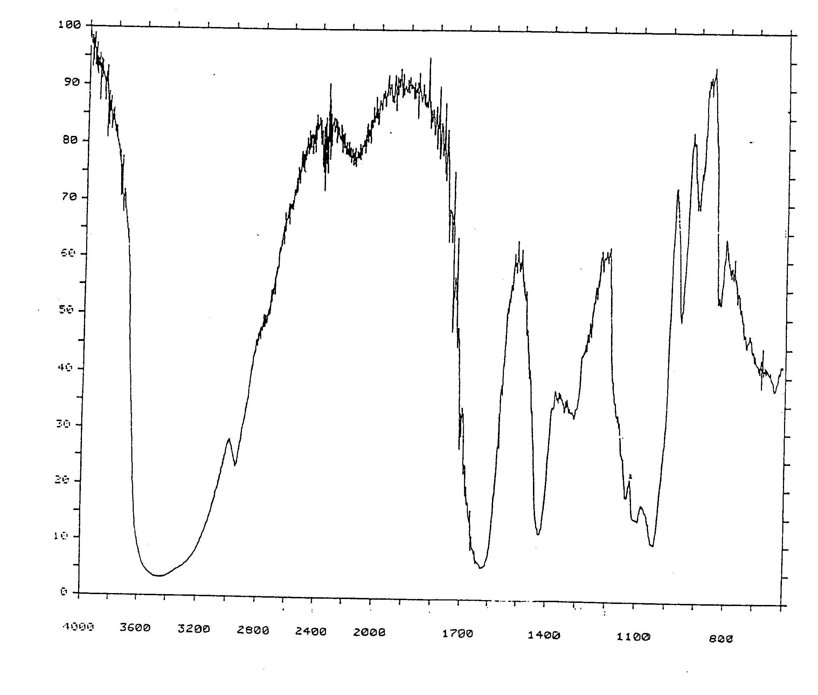

The analytical profile of the product obtained according to the process described in example 1 is shown in Table I. The infrared spectrum of said product is shown in Figure 1. The spectra have been determined on lozenges of potassium bromide with a 2% concentration of sample in a Perkin-Elmer model-881 infra-red spectrophotometer, scanning from 4000 to 600 cm$^{-1}$ at an interval of 6 minutes, with variable slit, resolution of 1.2 cm$^{-1}$ to 1000 cm$^{-1}$, and spectrum noise of 0.5 Y.T.; the spectra have been corrected electronically by the process of "smoothing", in accordance with the procedure of Savitzky/Golay, and have been expanded automatically in their absorbancy. It is possible to observe:
- Band centered at 1618 (1700 - 1500), corresponding to the C-0 tension of carboxyl radicals and carbonyl radical of strong intensity;
- Band centered at 1417 (1440 - 1395), characteristic of the carboxyl radical, C-0 tension, 0-H flexion of weak intensity, and the characteristic CH flexion of a strong aldehyde;
- Band at 1032 (1070 - 1000), characteristic of the C-0 tension of a strong hydroxyl radical.

Example 1:

- Preparing the suspension: As a first stage, a quantity of commercial alginic acid (Sigma Chemical Company), obtained from the alga Macrocystis pyrifera, is weighed to such effect that the concentration thereof in the final suspension will range between 5 and 25 mg/ml$^{-1}$, with the final volume comprising between 10 and 200 ml. Said quantity of alginic acid is suspended in distilled water through the instrumentality of manual agitation.
- Ultrasonic irradiation: As a second stage, said suspension is transferred to the receptacle of the ultrasound device, which latter is placed in the temperature-regulating appliance. Thereupon, the probe suitable for the volume is introduced, under the conditions described by the manufacturer. In the instance of this example, the suspension is subjected to ultrasound during one hour with an amplitude in the range of 5 - 15 μm and with a frequency of 23 KHz.
- Purification: As a third stage, the ultrasonically treated solution is transferred to a Visking dialysis bag with a molecular cut-off of from 10000 to 14000 daltons, said solution having been previously washed with water at 45º - 60º C and with cold distilled water, and it is dialyzed for 48 hours at 4º - 8º C against a volume of distilled water amounting to between 80 and 120 times the volume of dialyzed liquid, in the course of which changes of the dialysis liquid are effected every 24 hours.
- Drying: As a fourth stage, the dialyzed solution is dried by means of lyophilization.

## TABLE-I

Intrinsic Viscosity ....................... 3.7

Total Sugars (1), % by weight ............... 30

Total proteins (2), % by weight ............ 0

Free Carboxylates (3), % by weight ........ 13

Molecular Weight (4) ....................... $0.5-1.5 \ 10^6$ d

(1) - Determined in accordance with Dubois' method:
Dubois, A.: Gilles, K.A.; Hamilton, J.K.; Rebers, P.A.; and
Smith, F. (1956) Anal. Chem. 28:350-356. "Colorimetric
method for the determination of sugars and related
substances".

(2) - Determined in accordance with Lowry's method:
Lowry, O.H.; Rosenbrough, H.J.; Farr, A.L.; and Randall,
R.J. (1951) J. Biol. Chem. 193:265-275. "Protein measure-
ment with the folin phenol reagent".

(3) - Determined in accordance with Casu's method:
Casu, B.; and Gennaro, U. (1975) Carbohyd. Res. 39:168-176.
"A conductimetric method for the determination of sulphate
and carboxyl groups in heparin and other mucopolysaccha
rides".

(4) - Determined by molecular permeation with high-efficacy
liquid chromatography and with the use of commercial
dextrans in the range of 10000-700000 daltons as standards
of molecular weight.

## Claims

1.- Process for the obtaining of polymers with activity on the hematopoietic systems, characterized in that:
a) natural polycarboxylic polysaccharides are employed as starting products; b) suspensions having a definite concentration of the starting compounds are prepared in water; c) said suspensions are subjected to a process of ultrasonic irradiation under well-defined conditions of time, temperature, frequency and amplitude of the irradiation; d) once the process of ultrasonic irradiation has been completed, one proceeds to purify the resultant product, which is pharmacologically active, by means of a process of exhaustive dialysis under well-defined conditions of time, temperature, dialysis membrane and dialysis liquid; e) one then proceeds to obtain the pharmacologically active polysaccharide in the form of a solid by drying techniques such as lyophilization or pulverization/atomization of the dialyzed liquid; the polysaccharide in the form of a solid may be administered either directly as such or in suitable galenical forms; f) alternatively, the dialyzed liquid may be utilized, after quantitative determination of the pharmacologically active polysaccharide, for preparation of suitable galenical forms or for its direct use,

2.- Process for the obtaining of polymers with activity on the hematopoietic system, according to Claim 1, characterized in that, among the polycarboxylated polysaccharides which are employed as starting products, preference is given to alginic acid or the derivatives thereof, to gum arabic or to gum tragacanth.

3.- Process for the obtaining of polymers with activity on the hematopoietic system, according to Claims 1 and 2, characterized in that the sources of alginic acid or of its derivatives are algae such as <u>Laminaria digitata</u>, or <u>Macrocystis pyrifera</u>, or bacteria such as <u>Pseudomonas aeruginosa</u>, <u>Azotobacter vinelandii</u> or <u>Azotobacter chroococcum</u>.

4.- Process for the obtaining of polymers with activity on the hematopoietic system, according to Claims 1 and 2, characterized in that the sources of gum arabic are the various species of acacia (acacia trees of the leguminosae family), as, for example, <u>Acacia senegal</u> or <u>Acacia verek</u>.

5.- Process for the obtaining of polymers with activity on the hematopoietic system, according to Claims 1 and 2, characterized in that the sources of gum tragacanth are the various species of Astragalus of the leguminosae family, such as, for example, <u>Astragalus gummifer</u> Labill.

6.- Process for the obtaining of polymers with activity on the hematopoietic system, according to Claim 1, characterized in that the initial suspension contains the starting product in a concentration of between 5 and 25 mg per ml of distilled water.

7.- Process for the obtaining of polymers with activity on the hematopoietic system, according to Claim 1, characterized in that the time of subjection to ultrasonic irradiation ranges between 0.5 and 8 hours; in that the temperature of the suspension during the ultrasonic irradiation is maintained between 0° and 15° C; and in that the frequency of the ultrasonic irradiation is between 21 and 25 KHz, and the amplitude is between 5 and 15 μm.

8.- Process for the obtaining of polymers with activity on the hematopoietic system, according to Claim 1, characterized in that the products obtained after execution of the steps described above have a stimulant and restorative effect upon hematopoiesis, present little or no toxicity, and are suitable for the formulation of pharmaceutical preparations in the galenical forms appropiate for their use in human or veterinary medicine.